(19) Europäisches Patentamt

European Patent Office

Office européen des brevets

(11) **EP 2 645 092 A1**

(12) **EUROPEAN PATENT APPLICATION**

(43) Date of publication:
**02.10.2013 Bulletin 2013/40**

(51) Int Cl.:
*G01N 27/24* (2006.01)    *A23L 3/005* (2006.01)
*A23L 3/26* (2006.01)    *C12N 13/00* (2006.01)

(21) Application number: **12305391.0**

(22) Date of filing: **30.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Claranor**
**84140 Montfavet (FR)**

(72) Inventor: **Obin, Arnaud**
**78660 PARAY-DOUAVILLE (FR)**

(74) Representative: **Pontet Allano & Associes**
**Parc Orsay Université**
**25, rue Jean-Rostand**
**91893 Orsay Cedex (FR)**

(54) **Device comprising a window for transmitting radiation and an electrically conductive track arranged on the window for electrical detection of a break of the window**

(57)    Device (1) comprising: a window (2) (typically a quartz window) arranged for transmitting a radiation (typically UV radiation) in a given range of wavelengths through a transmitting surface (11) of the window; and a conductive track (5), the conductive track comprising a layer arranged for conducting electricity between a first electrical connection (9) and a second electrical connection (10) of the track, and being carried by the transmitting surface (11).

The invention also concerns a device for the decontamination of objects, comprising a transmitting device (1) according to the invention.

The application also concerns the corresponding methods.

FIG. 1

EP 2 645 092 A1

## Description

**Field of the invention**

The invention relates to:

- a device for transmitting radiation, comprising a window arranged for transmitting radiation, and
- a decontamination device comprising such a transmitting device.

It also relates to the corresponding methods.

The field of the invention is typically the decontamination of containers for the packaging in food and drugs industries by using UV radiations passing through a breakable window.

**Background of the invention**

The decontamination of containers by application of pulsed or continuous UV radiations is a well-known technique, widely used in particular in the food and medical industry.

The technique exploits the ability of UV radiations to destroy cells and microorganisms, which is essentially due to two effects: a photochemical effect and a photothermal effect.

The photochemical effect, which is obtained with pulsed and continuous UV radiations, results from the absorption by the microorganisms of the UV radiation. It causes breaches and formation of abnormal bonds in the DNA molecules which prevent proper DNA replication. The microorganism's protein production and cell metabolism is blocked and it dies.

The photothermal effect is obtained with pulsed radiation (pulsed light), and results from the delivery to the microorganism of a high energy in a very short time. The radiations absorbed by the microorganisms causes a steep increase of the internal temperature and breaches of the cells membranes, without heating much the environment.

Decontamination by UV radiations is in particular used in the food industry for the decontamination of containers for food products, such as bottles and caps, prior to their filling. This decontamination is necessary for the good preservation of the packaged products.

The containers are exposed to a UV radiation in the production line, to destroy to the desired extent or level of decontamination the microorganisms on their surfaces.

We know from prior art different methods for sterilizing containers and caps using UV radiations. In such methods, the UV radiations must typically go through a quartz window before reaching the containers and caps.

One problem with these techniques of decontamination by exposure to UV radiations is that the quartz window can be accidently broken for example:

- mechanically (vibration of the quartz window, shock of a decontaminated object on the window, ...), or
- due to a thermal stress (the temperature of the quartz window becomes too high)

This problem is critical, because small parts of the broken window can thus fall in the decontaminated container or cap. For example, in the food industry, any small parts of quartz should be absolutely avoided in a container containing food.

It is an object of the invention to provide a device for transmitting radiation, comprising a window arranged for transmitting radiation which brings a solution to the previously mentioned problem.

It is still another object of the invention to provide a decontamination device comprising such a transmitting device.

**Summary of the invention**

Such objects are accomplished with a transmitting device, comprising:

- a window arranged for transmitting a radiation in a given range of wavelengths through a transmitting surface of the window, and
- a conductive track, preferably in contact with said window, the conductive track comprising a layer (preferably a metallic layer) arranged for conducting electricity between a first electrical connection and a second electrical connection of the track, and being carried by the transmitting surface.

The transmitting device according to the invention can further comprise:

- means for electrically powering the track between the first electrical connection and the second electrical connection,
- and preferably also means for detecting a break of the electrical conductivity of the track.

The track can further be an optical mirror arranged for reflecting the radiation in the given range of wavelengths.

The track can comprise:

- an optional adhesion layer (typically made of chrome) in contact with the window,
- a conductive layer of a conductive materiel (a metal, typically aluminum) preferably in contact with the adhesion layer or in contact with the window if there isn't any adhesion layer.

The window can be covered by a protective layer (typically of silicon oxide) recovering the window and the track.

The means for detecting a break of the electrical conductivity can comprise means for measuring data depending on an electrical resistance of the conductive track. The transmitting device according to the invention can further comprise means for obtaining, based on this measured data or electrical resistance, a temperature of the window and/or a pollution state of the window and/or a recommendation for cleaning the window.

The conductive track can comprise three track parts electrically linked in series between the two connections:

- the first connection being linked in series with a track part to go,
- the track part to go being linked in series with a track part having a shape of a U-turn,
- the U-turn track part being linked in series with a return track part, the return track part being linked in series with the second connection.

Each one of the track part to go and the return track part can have:

- a shape of a periodic wave with many bends or turns, or
- a spiral shape, or
- any other possible shape allowing to explore widely the surface of the transmitting surface.

At any point of the return track part, the shortest distance between the track part to go and the return track part is preferably less than three times a width of the conductive track.

The track part to go and the return track part are preferably at a constant distance one to the other.

The transmitting surface is preferably a plane surface.

The window can comprise two opposite main surfaces. These main surfaces are preferably the largest surfaces of the window. The transmitting surface is preferably one of those main surfaces.

The conductive track can have a shape of a periodic wave or a spiral shape.

The window is preferably a quartz window.

An other aspect of the invention concerns a transmission method, comprising:

- transmitting a radiation in a given range of wavelengths through a transmitting surface of a window,
- conducting electricity between a first electrical connection and a second electrical connection of a conductive track (this track being preferably in contact with said window), the conductive track comprising a layer (preferably a metallic layer) arranged for conducting electricity between the first electrical connection and the second electrical connection, and being carried by the transmitting surface.

The transmission method according to the invention can further comprise:

- electrically powering the track between the first electrical connection and the second electrical connection, and
- detecting a break of the electrical conductivity of the track, preferably by measuring data depending on an electrical resistance of the conductive track; it can further comprise calculating and/or obtaining a temperature of the window and/or a pollution state of the window and/or a recommendation for cleaning the window, based on this resistance measurement.

The transmission method according to the invention can further comprise reflecting the radiation by the track which is further an optical mirror.

An other aspect of the invention concerns a device for the decontamination of objects, comprising:

- a decontamination zone for positioning at least one object,
- exposure means comprising at least a radiation source arranged for emitting a radiation, and optionally a reflector oriented toward the decontamination zone,

**characterized in that** it further comprises a transmitting device according to the invention and in that said exposure means are arranged for exposing to the radiation the at least one object into the decontamination zone through the transmitting surface of the window.

The transmitting device is preferably separating the exposure means from the decontamination zone, and the transmitting surface is preferably on the side of the exposure means.

An other aspect of the invention concerns a method for the decontamination of objects, comprising:

- positioning at least one object in a decontamination zone
- emitting a radiation with exposure means comprising at least a radiation source, and optionally a reflector oriented toward the decontamination zone **characterized in that** it further comprises:
- exposing to the radiation the at least one object into the decontamination zone through the transmitting surface of the window of a transmitting device according to the invention.

**Detailed description of the invention**

The devices and methods according to embodiments of the present invention may be better understood with reference to the drawings, which are given for illustrative purposes only and are not meant to be limiting. Other aspects, goals and advantages of the invention shall be apparent from the descriptions given hereunder.

- figure 1 shows a top view of window 2 and a track 5 of a device 1 for transmitting radiation 33 according to the invention (best realization mode), connected to a schematic view of electronic means 12, 13.
- figure 2 is a more detailed schematic view of the electronic means of the transmitting device of figure 1,
- figure 3 is a zoomed view of the window 2 and track 5 of figure 1,
- figure 4 shows a schematic profile cut view (according to plan I of figure 1) of a part 3 of the transmitting device of figure 1,
- figure 5 shows a schematic profile cut view of a decontamination device 4 according to the invention (best realization mode), comprising the transmitting device 1 according figure 1,
- figure 6 shows, compared to figure 5, a schematic profile cut view of a variant of a decontamination device 4 according to the invention, comprising the transmitting device 1 according figure 1, and
- figure 7 shows a top view of window 2 and a track 5 of a device 1 for transmitting radiation 33 according to the invention.

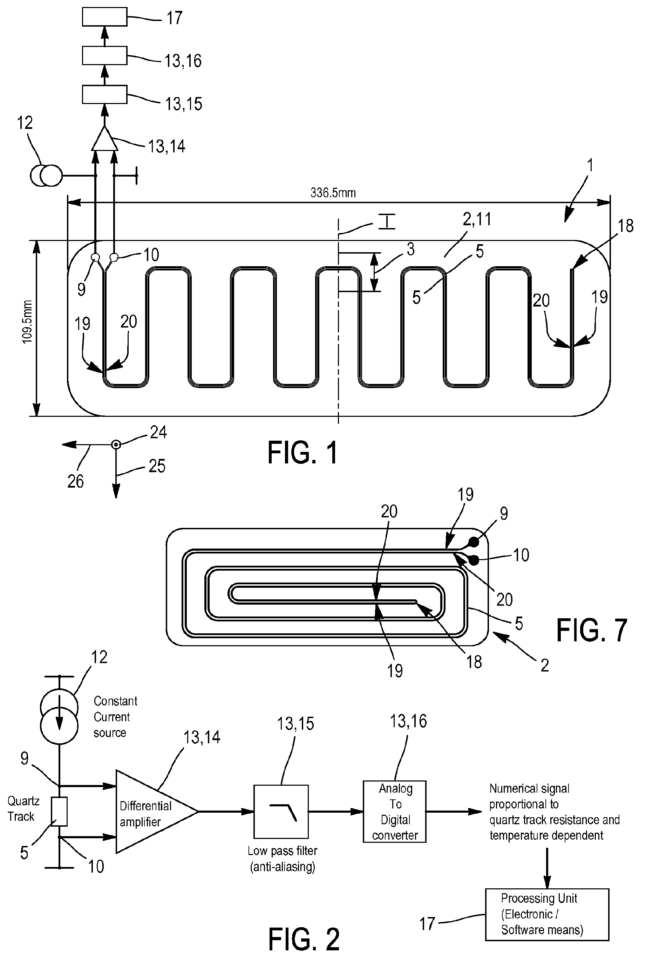

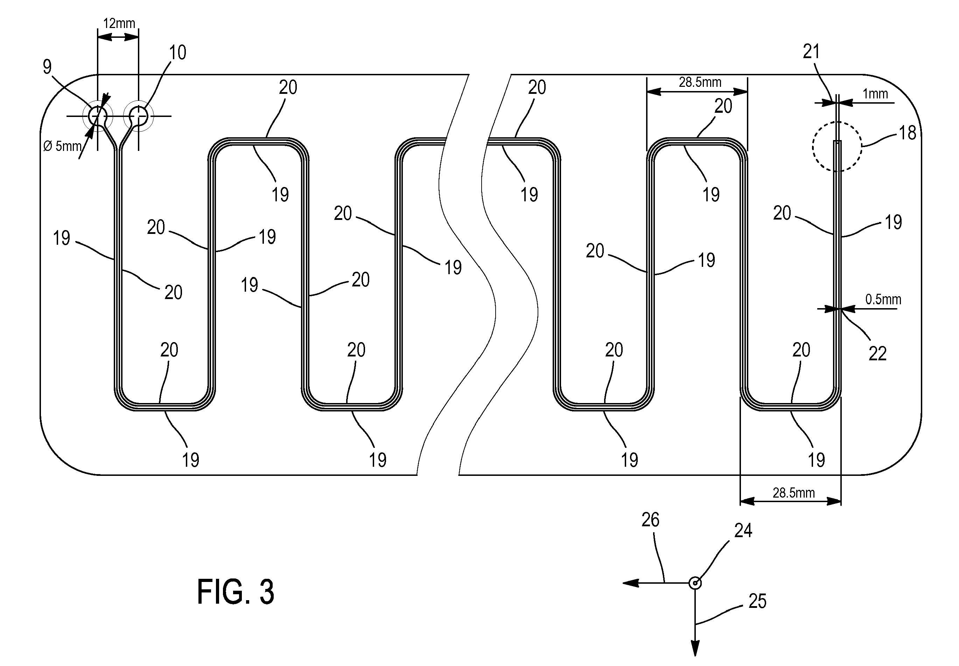

Typical dimensions in millimeters (mm) are given in figures 1, 3 and 4.

These embodiments being in no way limitative, we can consider variants of the invention including only a selection of the characteristics subsequently described, isolated from other described characteristics (even if this selection is taken from a sentence containing these other characteristics), if this selection of characteristics is sufficient to give a technical advantage or to distinguish the invention over the state of the art. This selection includes at least one characteristic, preferably a functional characteristic without structural details, or with only a part of the structural details if that part is sufficient to give a technical advantage or to distinguish the invention over the state of the art.

With reference to figures 1 to 4, the illustrated device 1 for transmitting radiation 33 according to the invention comprises :

- a window 2 arranged for transmitting radiation 33 (this radiation being light, preferably UV light) in a given range of wavelengths; the window 2 is typically a quartz window, and
- a conductive track 5 in contact with said window 2, the conductive track 5:

  • comprising at least a metallic layer 7 arranged for conducting electricity between a first electrical connection 9 and a second electrical connection 10 of the track 5, and
  • being carried by a transmitting surface 11 of the window 2.

The window 2 arranged for transmitting radiation 33 is arranged for transmitting at least 85%, preferably at least 90%, more preferably at least 99% of the intensity of radiation 33 in the given range, more preferably of any wavelength in the given range, and through the two main surfaces 11, 23 (from its main surface 11 to its other main surface 23).

The given range corresponds to a range of ultra violet (UV) wavelengths, preferably from 200 nm to 400 nm, more preferably from 190 nm to 280 nm.

The transmitting surface 11 is a plane surface. The transmitting surface 11 extends along two perpendicular axis 25, 26. Thickness is defined along axis 24 which is perpendicular to axis 25 and 26.

Window 2 comprises two opposite main surfaces 11 and 23, these main surfaces 11, 23 being preferably plane, and preferably the largest surfaces of window 2 in terms of area. The transmitting surface 11 is one of those main surfaces.

The transmitting device 1 further comprises:

- means 12 for electrically powering the track 5 between the first electrical connection 9 and the second electrical connection 10, these means comprising typically a constant current source, and
- means 13 for detecting a break of the electrical conductivity of the track 5. Means 13 comprise means for measuring data depending on an electrical resistance of the conductive track 5. These means 13 typically comprise an amplifier 14, a low pass filter 15, and an analog to digital converter 16.
- means 17 for calculating and obtaining a temperature of the window 2 and/or a pollution state of the window 2 and/or recommendations for cleaning the window 2. These means 17 typically comprise a processing unit 17 (comprising electronic and/or computer and/or software means)

An electronic interface comprising means 12, 13 is associated with track 5.

The electronic interface powers the track 5 with the constant current source 12 (2.5 mA is enough to avoid the undesirable electromagnetic field). The generated voltage is amplified by amplifier 14, and filtered by filter 15. The obtained signal is proportional to the electrical resistance $R_o$ of the track 5.

If track 5 is not broken, the obtained value of resistance $R_o$ is equal to a theoretical reference value of the resistance $R_{ref}$ (for a given temperature $T_{ref}$) of track 5 (typically between 10 and 1000 Ohms, typically around 400 Ohms), for example :

$$R_{ref} = r * L / S \text{ where :}$$

  r is the resistivity of aluminum (Ohm. Meter) at reference temperature $T_{ref}$
  L is the total length of track 5
  S is the section of the conductive layer 7

If the quartz window 2 is broken, then the track 5 should also be broken. The track 5 (and thus window 2) is detected broken when the track resistance $R_o$ increases too much compared to $R_{ref}$ (according to Ohm law the voltage increase when the impedance increases), for example if:

$$R_o > \beta \ R_{ref}$$

$\beta$ being a positive threshold coefficient, typically $100 < \beta$ (more preferably $1000 < \beta$ and/or $\beta < 10^7$, typically $\beta = 10000$

If $R_o < \beta \ R_{ref}$ and $R_o \neq R_{ref}$, the track 5 is not broken, but its resistance changed due to a temperature change. The temperature $T_o$ of track 5 is considered to be also the temperature of window 2.

$$As: \ R_o = R_{ref} + DeltaR$$

with deltaR = deltaT° x $\Omega$ x $R_{ref}$
and $\Omega$ is given in °C$^{-1}$ and is the temperature coefficient of resistance for the conductor material of layer 7. For Aluminium, $\Omega$=0.0043 °C$^{-1}$
and deltaT° = $T_o$ - $T_{ref}$ (typically $T_{ref}$=20°C)
thus $T_o$ is calculated by unit 17 based on $R_o$ as follows :

$$T_o = T_{ref} + (R_o - R_{ref}) / (\Omega \ R_{ref})$$

The temperature of window 2 increases if dirt and dust are sticking on it. Typically, dirt and dust are coming from the objects 29 decontaminated by radiation 33 transmitted through window 2. These dirt and dust absorb at least partially radiation 33, and this can heat window 2 or even break window 2.

$$If \ T_o - T_{ref} > \mu$$

$\mu$ being a positive temperature threshold value, typically 20°C$\leq\mu$ (more preferably 30°C$\leq\mu$) and/or $\mu \leq$100°C , typically $\mu$ = 20 or 30°C then unit 17 deduces (and displays on a screen):

- a pollution state of window 2, and
- a recommendation asking to a user to clean the window 2.

For example, for $T_{ref}$ = 20°C:

- if $T_o$ - $T_{ref} \leq \mu$ = 20°C or 30°C ($T_o \leq$ 40°C or 50°C), this corresponds to a normal use of window 2,
- if $T_o$ - $T_{ref} > \mu$ = 20°C or 30°C ($T_o >$ 40°C or 50°C), the pollution state of the window is "dirt" and unit 17 displays "you should clean the window", and
- if $T_o$ - $T_{ref} > \mu_2$= 60°C or 70°C ($T_o >$ 80°C or 90°C), the pollution state of the window is "very dirt" and unit 17 displays "it is now urgent to clean the window. The window is too hot and may be damaged".

Window 2 is typically cleaned with a solution of water and 70% ethanol and an optical cloth.

In order to be almost sure that a break of the quartz window 2 will lead to a break of the track 5, the track 5 should be distributed as much as possible over the transmitting surface 11 of the window 2. The conductive track 5 has a shape of a periodic wave, that preferably extends substantially uniformly over the surface 11. Nevertheless, this is not obvious to do that, because of the following area problem: if the track 5 occupies an important part of the area of the transmitting surface 11, then the track 5 would prevent the radiation 33 to be transmitted through the window 2.

In order to solve this area problem, the track 5 is further an optical mirror. This mirror comprises the at least one metallic layer 7 that reflects radiation 33. This optical mirror is arranged for reflecting radiation 33, at least 85%, preferably at least 90% of the intensity of the radiation 33 in the given range, more preferably of any wavelength in the given range.

The thickness of window 2 is 3 millimeters.

Track 5 comprises at least two layers:

- An optional adhesion layer 6 (of chromium) evaporated directly on the quartz window 2, preferably for sticking track 5 on the quartz window 2; the thickness of this layer 6 is 80 nanometers;
- the conductive layer 7 of electrically conductive material (a metal, typically aluminum in this particular embodiment), realized preferably directly on the adhesion layer 6 or directly on the quartz window 2 if there isn't any adhesion layer 6, for the track 5 illustrated in figure 1, the thickness of layer 7 is 650 nanometers so that $R_{ref}$ is around 400 Ohms.

A protection layer 8 of silicon oxide $SiO_2$ is realized over the entire transmitting surface 11 (expected on connections 9, 10), and is realized preferably directly on the quartz window 2 or directly on the track 5 where track 5 is present. In other variants, the protection layer 8 can be made of $MgF_2$ or $Al_2O_3$. The thickness of this layer 8 is typically around 150 nanometers.

Thus, metallic layer 7 is used for radiation reflection and also for electrical conduction.

This way, radiation energy is not lost by absorption by track 5, but radiation 33 is reflected by track 5 in order to be reflected by another reflector 17 in order to try again to be transmitted through window 2.

The conductive track 5 comprises three track parts electrically linked in series between the two connections 9, 10:

- the first connection 9 is linked in series with a track part to go 19,
- the track part to go 19 is linked in series with a track part 18 having a shape of a U-turn,
- the U-turn track part 18 is linked in series with a return track part 20, the return track part 20 being linked in series with the second connection 10.

The track part to go 19 and the return track part 20 are parallel i.e. at a constant distance 21 one to the other. This way, the return track part 20 "follows" the track part to go 19. This allows to minimize the internal area, comprised between the track part to go 19 and the return track part 20, of the electrical loop formed by track 5. This way, it allows to increase the electromagnetic immunity of the device according to the invention and thus to avoid measuring parasitic signals. This way, it increases the precision for measuring $R_o$ and $T_o$. Furthermore, at any point of the return track part 20, the shortest distance 21 between the track part to go 19 and the return track part 20 is inferior to three times the width 22 of the conductive track 5 and has preferably a constant value. The width 22 of track 5 is defined along axis 26 when track 5 is extending along axis 25, and is defined along axis 25 when track 5 is extending along axis 26.

The conductive track 5 has a shape of a periodic wave, that preferably extends substantially uniformly over the surface 11. Both the track part to go 19 and the return track part 20 have this shape of a periodic wave.

Figure 7 shows a top view of window 2 of a variant of the device 1 previously described. The only difference between window 2 of figure 7 and window 2 of figure 1 is that window 2 according to figure 7 has a track part to go 19 and a return track part 20 having a spiral shape. This illustrates that the parts 19, 20 can have various possible shapes.

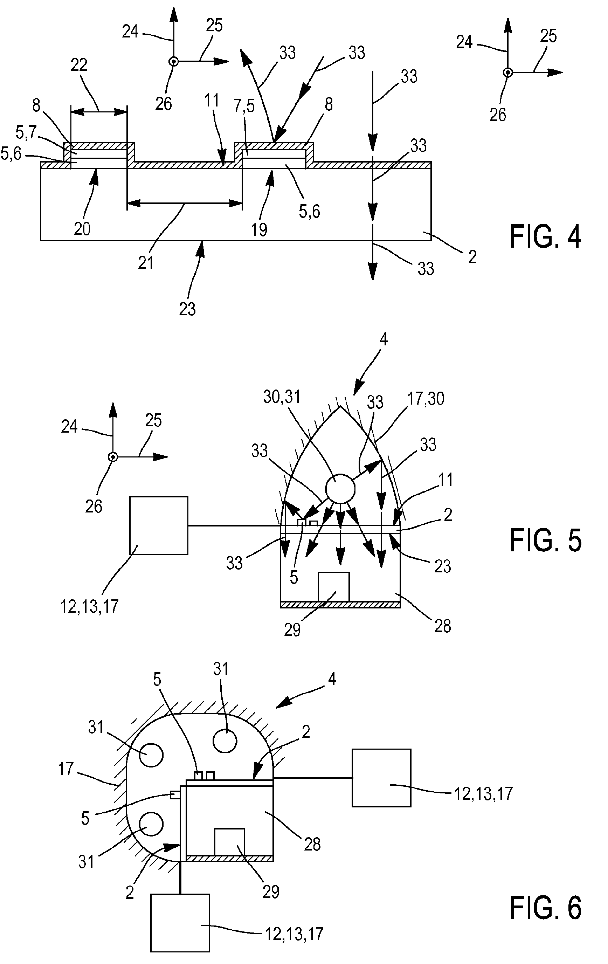

With reference to figure 5, the previously illustrated device 1 for transmitting radiation 33 can be integrated in a device 4 for the decontamination of objects according to the invention.

The decontamination device 4 comprises:

- a decontamination zone 28 for positioning objects 29
- exposure means 30 comprising at least an UV radiation source 31 (arranged for emitting the UV radiation 33 in the given range of wavelength) and a reflector 17 oriented toward the decontamination zone 28,

Radiation 33 comprises several wavelengths in the given range of wavelengths.

The decontamination zone 28 has an elongated shape in a direction of elongation 26, and is arranged for containing a plurality of objects 29.

Each UV radiation source 31 comprises at least one Xenon flash lamp or one Eximer lamp. Each radiation source 31 can further emit other radiations out of the given range.

Decontamination device 4 further comprises transmitting device 1.

Window 2 is physically separating exposure means 30 and the decontamination zone 28.

Exposure means 30 are arranged for exposing to the UV radiation 33 objects 29 into the decontamination zone 28 through the transmitting surface 11 of window 2, more precisely through the two main surfaces 11, 23 of window 2.

Among the two main surfaces 11, 23, the transmitting surface 11 is facing the exposure means 30 and the other main surface 23 is facing the decontamination zone 28. This avoids any damages caused to track 5 by objects 29.

The method for decontaminating objects 29 in device 4 comprises the following steps:

- positioning at least one object 29 in the decontamination zone 28,
- emitting UV radiation 33 with exposure means 30,
- exposing to the UV radiation 33 the objects 29 into the decontamination zone 28 through the transmitting surface 11 of window 2.

Said exposing step comprises the following steps of a method for transmitting radiation 33 according to the invention:

- transmitting radiation 33 in the given range of wavelengths through window 2, and
- conducting electricity between the connection 9, 10 of a track 5 in contact with said window 2 and carried by transmitting surface 11.

It further comprises:

- electrically powering the track 5 between the first electrical connection 9 and the second electrical connection 10, and
- detecting a break of the electrical conductivity of the track 5, preferably by measuring data depending on an electrical resistance of the conductive track 5;

It can further comprises calculating and obtaining the temperature of the window 2 and/or the pollution state of the window 2 and/or recommendations for cleaning the window 2.

It further comprises reflecting, towards exposure means 30 (more precisely towards reflector 17), radiation 33 by track 5 which further plays the function of an optical mirror.

As illustrated in Figure 6, the device 4 can comprise:

- more than one device 1, and/or

- More than one radiation source 31,
- etc..;

While this invention has been described in conjunction with a number of embodiments, it is evident that many alternatives, modifications and variations would be or are apparent to those of ordinary skill in the applicable arts. Accordingly, it is intended to embrace all such alternatives, modifications, equivalents and variations that are within the spirit and scope of this invention. For example, window 2 is not necessary made of quartz.

Of course, the invention is not limited to the examples which have just been described and numerous amendments can be made to these examples without exceeding the scope of the invention.

Of course, the different characteristics, forms, variants and embodiments of the invention can be combined with each other in various combinations. In particular all variants and embodiments described above can be combined with each other.

**Claims**

1. A transmitting device (1), comprising:

   - a window (2) arranged for transmitting a radiation (33) in a given range of wavelengths through a transmitting surface (11) of the window, and
   - a conductive track (5), the conductive track comprising a layer (7) arranged for conducting electricity between a first electrical connection (9) and a second electrical connection (10) of the track, and being carried by the transmitting surface.

2. The transmitting device according to claim 1, **characterized in that** the track is further an optical mirror arranged for reflecting the radiation (33) in the given range of wavelengths.

3. The transmitting device according to claim 1 or 2, **characterized in that** it further comprises:

   - means (12) for electrically powering the track between the first electrical connection and the second electrical connection, and
   - means (13) for detecting a break of the electrical conductivity of the track.

4. The transmitting device according to claim 3, **characterized in that** the means (13) for detecting a break of the electrical conductivity comprise means (14, 15, 16) for measuring data depending on an electrical resistance of the conductive track.

5. The transmitting device according to claim 4, **characterized in that** it further comprises means (17) for obtaining, based on this measured data or electrical resistance, a temperature of the window and/or a pollution state of the window and/or a recommendation for cleaning the window.

6. The transmitting device according to any one of the previous claims, **characterized in that** the conductive track comprises three track parts electrically linked in series between the two connections, the first connection being linked in series with a track part to go (19), the track part to go being linked in series with a track part (18) having a shape of a U-turn, the U-turn track part being linked in series with a return track part (20), the return track part being linked in series with the second connection.

7. The transmitting device according to claim 6, **characterized in that**, at any point of the return track part, the shortest distance (21) between the track part to go and the return track part is less than three times a width (22) of the conductive track.

8. The transmitting device according to claim 6 or 7, **characterized in that** the track part to go and the return track part are at a constant distance (21) one to the other.

9. The transmitting device according to any one of the previous claims, **characterized in that** the transmitting surface is a plane surface.

10. The transmitting device according to any one of the previous claims, **characterized in that** the window comprises two opposite main surfaces (11, 23), these main surfaces being the largest surfaces of the window, the transmitting surface being one of those main surfaces.

11. The transmitting device according to any one of the previous claims, **characterized in that** the conductive track has a shape of a periodic wave or has a spiral shape.

12. A transmission method, comprising:

   - transmitting a radiation (33) in a given range of wavelengths through a transmitting surface (11) of a window (2),
   - conducting electricity between a first electrical connection (9) and a second electrical connection (10) of a conductive track (5), the conductive track comprising a layer (7) arranged for conducting electricity between the first electrical connection and the second electrical connection, and being carried by the transmitting surface.

**EP 2 645 092 A1**

13. A device for the decontamination of objects, comprising:

> - a decontamination zone (28) for positioning at least one object (29),
> - exposure means (30) comprising at least a radiation source (31) arranged for emitting a radiation (33),
> **characterized in that** it further comprises a transmitting device (1) according to any one of claims 1- 11 and **in that** said exposure means are arranged for exposing to the radiation (33) the at least one object into the decontamination zone through the transmitting surface (11) of the window.

14. The decontamination device according to the previous claim, comprising the transmitting device according to claim 10, **characterized in that** the transmitting device is separating the exposure means from the decontamination zone, and **in that**, among the two main surfaces, the transmitting surface is on the side of the exposure means and the other main surface is on the side of the decontamination zone.

15. A method for the decontamination of objects, comprising:

> - positioning at least one object (29) in a decontamination zone (28),
> - emitting a radiation (33) with exposure means (30) comprising at least a radiation source (31),
> **characterized in that** it further comprises:
> - exposing to the radiation (33) the at least one object into the decontamination zone through the transmitting surface (11) of the window of a transmitting device (1) according to any one of claims 1-11.

FIG. 1

FIG. 7

336.5mm

109.5mm

Constant
Current
source

Quartz
Track

Differential
amplifier

Low pass filter
(anti-aliasing)

Analog
To
Digital
converter

Numerical signal
proportional to
quartz track resistance and
temperature dependent

Processing Unit
(Electronic /
Software means)

FIG. 2

FIG. 3

FIG. 4

FIG. 5

FIG. 6

EP 2 645 092 A1

Europäisches
Patentamt
European
Patent Office
Office européen
des brevets

**EUROPEAN SEARCH REPORT**

Application Number

EP 12 30 5391

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 227 348 A (CHARLES DUNCAN HENRY WEBB) 7 April 1971 (1971-04-07) * the whole document * | 1-12 | INV. G01N27/24 A23L3/005 A23L3/26 C12N13/00 |
| X | DE 199 60 450 C1 (SAINT GOBAIN GLASS DEUTSCHLAND [DE]) 23 May 2001 (2001-05-23) * the whole document * | 1-12 | |
| A | US 2004/046127 A1 (WONG TOMMY CHI-KIN [HK]) 11 March 2004 (2004-03-11) * the whole document * | 13-15 | |

TECHNICAL FIELDS
SEARCHED    (IPC)

G01N
A23L
C12N
C12M

The present search report has been drawn up for all claims

| Place of search | Date of completion of the search | Examiner |
|---|---|---|
| Munich | 16 October 2012 | Klein, Marc-Oliver |

**ANNEX TO THE EUROPEAN SEARCH REPORT
ON EUROPEAN PATENT APPLICATION NO.**

EP 12 30 5391

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2012

| Patent document cited in search report | | Publication date | Patent family member(s) | | Publication date |
|---|---|---|---|---|---|
| GB 1227348 | A | 07-04-1971 | NONE | | |
| DE 19960450 | C1 | 23-05-2001 | AT | 271243 T | 15-07-2004 |
| | | | DE | 19960450 C1 | 23-05-2001 |
| | | | EP | 1109140 A2 | 20-06-2001 |
| | | | ES | 2223427 T3 | 01-03-2005 |
| | | | PT | 1109140 E | 30-11-2004 |
| | | | TR | 200402673 T4 | 22-11-2004 |
| US 2004046127 | A1 | 11-03-2004 | NONE | | |

EPO FORM P0459

For more details about this annex : see Official Journal of the European Patent Office, No. 12/82